# FASCICULE DE BREVET EUROPEEN

(11) **EP 0 627 909 B1**
(45) Date de publication et mention de la délivrance du brevet: **03.03.1999**
(21) Numéro de dépôt: 94903915.0
(22) Date de dépôt: 23.12.1993
(51) Int. Cl.: A61K 7/48

(54) **COMPOSITION COSMETIQUE OU PHARMACEUTIQUE CONTENANT EN ASSOCIATION UN POLYPHENOL ET UN EXTRAIT DE GINKGO**
KOSMETISCHE ODER PHARMAZEUTISCHE ZUSAMMENSETZUNG, DIE EINE KOMBINATION AUS EINEM POLYPHENOL UND GINKGO-EXTRAKT ENTHAELT
COSMETIC OR PHARMACEUTICAL COMPOSITION CONTAINING A COMBINATION OF A POLYPHENOL AND A GINKGO EXTRACT

(30) Priorité: 24.12.1992 FR 9215725
(43) Date de publication de la demande: 14.12.1994
(73) Titulaire: L'OREAL, 75008 Paris (FR)
(72) Inventeur: N'GUYEN, Quang-Lan, F-92160 Antony (FR)
(74) Mandataire: Tonnellier, Jean-Claude
(86) Numéro de dépôt international: FR9301295
(87) Numéro de publication internationale: WO9414414

(56) Documents cités:
- EP-A- 0 275 005
- EP-A- 0 307 626
- EP-A- 0 353 161
- EP-A- 0 496 173
- FR-A- 2 400 358
- FR-A- 2 667 505
- PARFUMS, COSMéTIQUES ET ARôMES vol. 96 , 1991 , PARIS (FRANCE) pages 77 - 86 JEAN MORELLE-ELIANE LAUZANNE 'que peuvent nous apporter les extraits végétaux?: le ginkgo biloba (salisburia adiantifolia)'
- CHEMICAL ABSTRACTS, vol. 116, no. 4, 27 Janvier 1992, Columbus, Ohio, US; abstract no. 28119h, K. MATSUI 'manufacture of extract with high content of flavonoids from gingko leaves' & JP,A,3 227 985 (ICHIMARU PHARCOS CO., LTD.) 8 Octobre 1991

## Description

L'invention a pour objet une composition cosmétique ou pharmaceutique contenant en tant qu'antioxydant une association à action synergique d'un extrait de ginkgo et d'au moins un dérivé polyphénolique.

La plupart des compositions cosmétiques ou pharmaceutiques sont constituées d'une phase grasse dont les produits huileux ont une certaine tendance à s'oxyder, même à température ambiante. Cette oxydation a pour conséquence d'en modifier profondément les propriétés, notamment olfactives, ce qui les rend inutilisables après une période de temps variable.

Afin de protéger les compositions vis-à-vis de ces phénomènes d'oxydation, il est de pratique courante d'incorporer des agents protecteurs qui jouent le rôle d'antioxydants.

Si les antioxydants sont particulièrement utiles pour la bonne conservation des corps gras des compositions cosmétiques ou pharmaceutiques, on sait maintenant que certains d'entre eux permettent également de lutter contre les effets nocifs des substances oxydantes, formées sous l'action des radicaux libres engendrés notamment par les polluants atmosphériques et les ultra-violets. Ces effets nocifs s'exercent en particulier sur les cellules de la peau et des muqueuses en contact avec le milieu extérieur.

Il est donc important de pouvoir disposer d'agents antioxydants capables d'inhiber la formation de radicaux libres et permettant de lutter contre des phénomènes d'oxydation pouvant provoquer des dommages cellulaires irréversibles.

Le document EP-A-0275005 décrit un complexe formé par la combinaison de flavonoïdes avec des phospholipides.

Le document Parfums, cosmétiques, arômes n° 96, 77-86 (1991) étudie les constituants de ginkgo biloba et divers extraits de ce végétal, sans décrire d'extraits obtenus avec des solvants non polaires.

On a maintenant découvert qu'il était possible à la fois d'obtenir une bonne conservation des compositions cosmétiques ou pharmaceutiques contenant des corps gras facilement oxydables et de protéger efficacement la peau ou les muqueuses en utilisant, en association, un extrait de ginkgo et au moins un composé polyphénolique. On a découvert en outre que, de façon surprenante, cette association possède des propriétés synergiques.

Par l'expression "composé polyphénolique" on entend les composés comportant au moins un cycle aromatique diphénolique, les groupement phénols pouvant être éventuellement éthérifiés ou estérifiés. Dans ce qui suit, un tel composé peut aussi être appelé simplement "polyphénol".

L'invention a donc pour objet une composition cosmétique ou pharmaceutique contenant un système anti-oxydant à action synergique constitué par l'association d'un extrait de ginkgo et d'au moins un composé polyphénolique.

La substance active (ou les substances actives) de l'extrait de ginkgo n'est pas connue, mais cette substance active peut être obtenue en procédant à une extraction de la matière végétale, et notamment des feuilles, à l'aide d'un solvant non polaire. On désigne ci-après par l'expression "extrait apolaire" soit un tel extrait, soit une ou plusieurs substances actives contenues dans un tel extrait et pouvant en être isolées par une purification plus poussée. Une substance active désigne ici une substance ayant une activité anti-oxydante (pouvant être mise en évidence par exemple selon un test d'auto-oxydation de la vitamine F tel que celui décrit dans la partie expérimentale ci-après) et dont l'association avec un polyphénol permet de mettre en évidence une action anti-oxydante synergique.

On utilise notamment un extrait de feuilles de ginkgo biloba.

L'extrait apolaire de ginkgo peut être obtenu par évaporation à sec des fractions issues de l'extraction avec un solvant apolaire de feuilles de ginkgo. Comme solvant non polaire on peut citer les alcanes linéaires, ramifiés ou cycliques en C₆-C₁₄, le n-hexane étant particulièrement préféré. De tels extraits sont décrits notamment dans le brevet japonais 91-014 007.

Les composés polyphénoliques utilisés dans la composition de l'invention peuvent être choisis parmi ceux qui présentent une activité anti-oxydante dans un test d'auto-oxydation de la vitamine F tel que celui décrit ci-après dans la partie expérimentale.

Le polyphénol peut être choisi par exemple parmi :
a) les flavonoïdes
b) l'acide carnosique ou le carnosol
c) les acides (2,5-dihydroxy phényl) carboxylique et (2,5-dihydroxy phényl)alkylène carboxylique, éventuellement substitués, et leurs dérivés, notamment leurs sels, esters ou amides,
d) les esters ou amides de l'acide caféique,
e) l'acide tannique.

Parmi les polyphénols utilisables, on citera notamment les flavonoïdes répondant à la formule générale (I) : 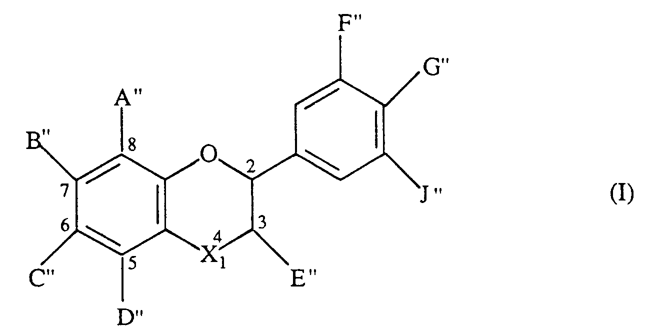 ou (II) : 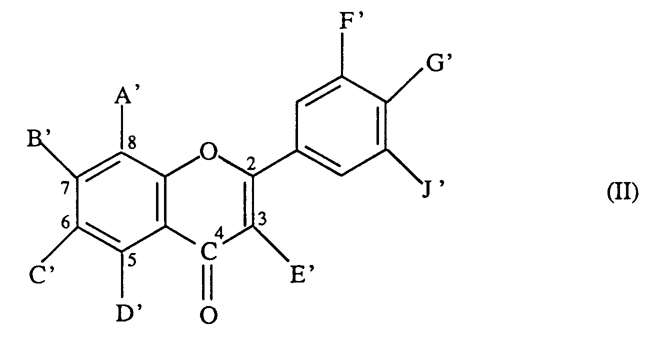 dans lesquelles A'', B'', C'', et D'', indépendamment l'un de l'autre, représentent H ou OH ; E'' représente H, OH ou OX', où X' représente : 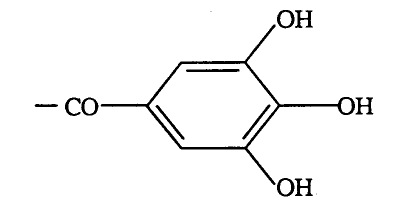 F'', G'', J'' représentent, indépendamment l'un de l'autre, H ou OH ; et X₁ représente -CH₂-, -CO- ou -CHOH-,
A', C' et D', indépendamment l'un de l'autre, représentent H, OH ou OCH₃; E' représente H, OH ou OR', où R' représente le reste d'un sucre de formule R'OH ; B', F', G' et J', indépendamment l'un de l'autre, représentent H, OH, OCH₃ ou -OCH₂-CH₂-OH. Parmi les sucres R'OH, on peut citer le rutinose.

Les composés de formule (I) et (II) sont connus. Ils peuvent être obtenus notamment selon les procédés décrits dans "The Flavonoids" Harborne J.B., Mabry T.J., Helga Mabry, 1975, pages 1 à 45.

Parmi les flavonoïdes utilisables selon l'invention, on citera notamment la taxifoline, la catéchine, l'épicatéchine, l'eriodictyol, la naringénine, la rutine, la troxérutine, la chrysine, la tangérétine, la lutéoline, l'épigallocatéchine et le gallate de l'épigailocatéchine, la quercétine, la fisétine, le kaëmpférol, la galangine, la gallocatéchine, le gallate d'épicatéchine.

Certains polyphénols utilisables sont présents dans des végétaux dont ils peuvent être extraits de façon connue. On peut utiliser des extraits de feuilles de thé (Camellia sinensis ou Camellia japonica). On citera en particulier les extraits de thé vert vendus sous la dénomination Sunphenon par la Société Nikko, qui contiennent notamment des flavonoïdes.

Parmi les polyphénols utilisables, on citera également des polyphénols tels que l'acide carnosique et le carnosol qui peuvent être extraits par exemple du romarin soit par extraction suivie d'une distillation (Chang et al. JOSC, Vol.61, n°6, Juin 1984), soit par une extraction par un solvant polaire tel que l'éthanol précédée par une extraction à l'aide d'un solvant non polaire tel que l'hexane pour éliminer les substances odorantes, comme décrit dans la demande de brevet EP-307 626.

Les polyphénols utilisables peuvent également être choisis parmi les acides (2,5-dihydroxyphényl)alkylcarboxyliques de formule (III) et leurs dérivés (notamment esters et amides) : 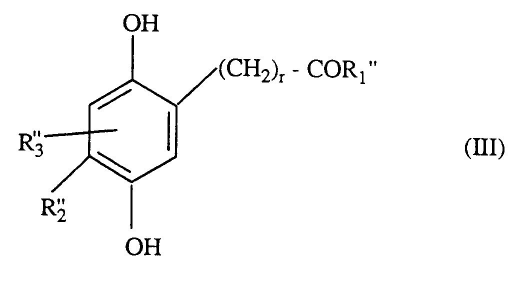 dans laquelle :
R''₁ représente -O-Alc, OH ou -N(r')(r''), Alc étant un alkyle linéaire ou ramifié en C₁-C₂₀, éventuellement substitué par un ou plusieurs groupements hydroxy ou alcoxy, ou Alc étant un alcényle en C₂-C₂₀,
r' et r'' représentent indépendamment H, alkyle C₁-C₂₀, hydroxyalkyle en C₂-C₆, ou polyhydroxyalkyle en C₃-C₆, ou bien r' et r'' forment ensemble, avec l'atome d'azote auquel ils sont rattachés, un hétérocycle,
r est un nombre, y compris zéro, tel que la chaîne -(CH₂)ᵣ-COR₁ comporte au plus 21 atomes de carbone,
R''₂ et R''₃ représentent indépendamment H ou un alkyle C₁-C₄, R''₂ pouvant représenter en outre un alcoxy C₁-C₄.

Les composés de formule (III) sont connus ou peuvent être préparés selon des méthodes connues, par exemple analogues à celles décrites dans les brevets FR-2.400.358 et FR-2.400.359.

Parmi les polyphénols utilisables selon l'invention, on citera également les esters ou amides de l'acide caféique. Parmi les esters de l'acide caféique, on peut mentionner notamment les composés de formule (IV) : 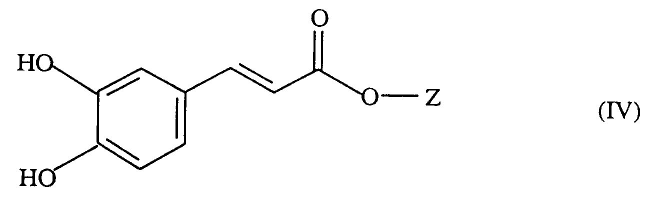 dans laquelle Z représente un alkyle C₁-C₈, par exemple méthyle, ou le reste d'un phytol.

Parmi les amides de l'acide caféique, on peut citer notamment les composés de formule (V) : 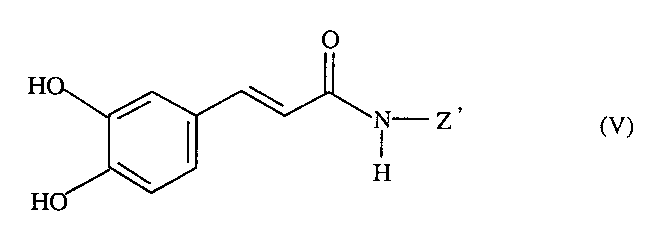 dans laquelle Z' représente un alkyle en C₁-C₈, en particulier en C₆-C₈.

Les composés de formule (IV) ou (V) sont connus ou peuvent être préparés selon les méthodes connues.

L'acide tannique est présent notamment dans l'extrait de noix d'Alep commercialisé sous la dénomination Supextrat par la Société Sochibo.

Dans les compositions cosmétiques ou pharmaceutiques selon l'invention, l'extrait apolaire de ginkgo est généralement présent à une concentration comprise entre 1 et 10 % en poids par rapport au poids total de la composition.

Le polyphénol tel que défini ci-dessus est présent en une proportion comprise entre 0, 1 et 1 % en poids par rapport au poids total de la composition.

Les proportions relatives optimales d'extrait apolaire de ginkgo et de polyphénol peuvent être déterminées pour chaque type de composition par de simples expériences de routine. Le rapport en poids entre l'extrait apolaire de ginkgo et le polyphénol est généralement compris entre 1 et 100, et en particulier voisin de 10.

Les compositions cosmétiques ou pharmaceutiques de l'invention peuvent contenir, outre l'association de principes actifs décrite ci-dessus, et un véhicule approprié, les ingrédients ou adjuvants habituellement utilisés dans la réalisation de telles compositions. Elles peuvent contenir en particulier des solvants tels que l'eau, des solvants organiques (par exemple alcools, huiles) ou des silicones, des agents épaississants, des agents tensioactifs, des polymères, des corps gras solides (par exemple cires, lanoline), des agents humectants, des agents conservateurs, des agents modificateurs de pH, des agents séquestrants, des agents colorants, des parfums, des charges solides (poudres et pigments), des substances absorbant l'ultra-violet, des agents autobronzants (comme la dihydroxyacétone), etc.

Les compositions sous forme de dispersions vésiculaires contiennent par exemple au moins un ingrédient actif incorporé dans des micelles ou des bicouches lipidiques, pouvant encapsuler une phase aqueuse, et dispersées dans un solvant aqueux.

Les dispersions vésiculaires de lipides, notamment de lipides amphiphiles ioniques ou non-ioniques, sont préparées selon des procédés connus, par exemple en faisant gonfler les lipides dans une solution aqueuse pour former des sphérules dispersées dans le milieu aqueux, comme décrit dans l'article de Banghan, Standish et Watkins, J. Mol. Biol. 13, 238 (1965) ou dans les brevets FR 2 315 991 et 2 416 008. On trouvera aussi la description de divers modes de préparation dans "Les liposomes en biologie cellulaire et pharmacologie", Edition INSERM/John Libbery Eurotext, 1987, pages 6 à 18.

Les compositions peuvent se présenter sous forme de dispersions de nanoparticules. Le terme "nanoparticules" recouvre d'une part les nanosphères et, d'autre part, les nanocapsules ; on désigne par le terme "nanosphères" les nanoparticules constituées par une matrice polymérique poreuse sur laquelle le principe actif est absorbé et/ou adsorbé et par le terme "nanocapsules", les nanoparticules constituées par une membrane polymérique, qui entoure un coeur formé par le principe actif. De telles formes de composition sont décrites par exemple dans les demandes de brevet EP-274 961 et FR-2 659 554.

Les compositions de l'invention sont notamment des compositions cosmétiques ou pharmaceutiques protectrices de l'épiderme humain, des cheveux et des muqueuses, des compositions de maquillage de la peau et des phanères, des compositions à usage bucco-dentaire telles que des pâtes dentifrices, ou des compositions ophtalmiques telles que des collyres.

Lorsque la composition cosmétique selon l'invention est utilisée pour la protection des cheveux, elle peut se présenter sous forme de shampooings, de lotions, de gels ou de compositions à rincer, à appliquer avant ou après shampooing, avant ou après coloration ou décoloration, ou avant, pendant ou après traitement de permanente ou de défrisage. Elle peut encore se présenter sous la forme de lotions ou de gels coiffants ou traitants, de lotions ou gels pour le brushing ou la mise en plis, de laques pour cheveux, de compositions de permanente ou de défrisage, ou de compositions de coloration ou de décoloration des cheveux.

Lorsque la composition de l'invention est utilisée comme produit de maquillage des cils, des sourcils ou de la peau, elle se présente par exemple sous la forme de crèmes de traitement de l'épiderme, de fonds de teint, de bâtons de rouge à lèvres, de fards à paupières, de fards à joue, de ligneurs (encore appelés "eye-liners") ou de mascaras.

Lorsque la composition de l'invention est une composition pharmaceutique, elle peut se présenter notamment sous forme d'émulsion (lait ou crème), de gel, de lotion, de pommade, de dispersion vésiculaire ou de dispersion de nanoparticules, et peut contenir, outre l'association décrite ci-dessus, un autre principe actif pharmaceutique.

Grâce à l'association synergique qu'elles contiennent, les compositions de l'invention constituent des compositions cosmétiques ou pharmaceutiques destinées à être appliquées notamment sur la peau, les phanères et les muqueuses, qui permettent par exemple de prévenir et traiter les dommages provoqués par les radicaux libres induits notamment par les polluants atmosphériques et par le rayonnement ultraviolet. En particulier, les compositions cosmétiques de l'invention permettent de prévenir ou de traiter le phénomène de vieillissement accéléré de la peau.

L'invention a également pour objet l'utilisation, en association, d'au moins un extrait de ginkgo et d'au moins un polyphénol, comme association active synergique dans la préparation d'une composition cosmétique ou pharmaceutique destinée à prévenir ou traiter les dommages cellulaires provoqués, sur la peau, le cuir chevelu ou les muqueuses, par les radicaux libres induits notamment par les polluants atmosphériques et/ou par le rayonnement ultraviolet, et/ou destinée à lutter contre le phénomène de vieillissement accéléré de la peau.

L'invention a également pour objet un procédé de traitement cosmétique permettant de lutter contre les dommages esthétiques provoqués sur la peau et les cheveux par les radicaux libres induits notamment par les polluants atmosphériques et par le rayonnement ultraviolet, caractérisé par le fait que l'on applique sur la peau ou les cheveux une composition contenant l'association synergique qui a été décrite ci-dessus.

Les exemples suivants illustrent l'invention.

Dans ces exemples, les extraits de ginkgo utilisés ont été préparés de la façon indiquée ci-après. L'extrait de thé vert "Sunphenon" est commercialisé par Nikko Chemicals. L'extrait hydroalcoolique de noix d'Alep est commercialisé par Sochibo sous la dénomination Supextrat.

### MODE D'OBTENTION DES EXTRAITS DE GINKGO

On introduit des feuilles de ginkgo biloba réduites à l'état de poudre végétale dans une cartouche en carton cellulosique poreux.

La cartouche est introduite dans un extracteur de type "soxhlet" : cet extracteur est équipé à sa base d'un ballon contenant l'hexane chauffé à ébullition (69°C). Les vapeurs de solvant passent par une dérivation, se condensent dans le réfrigérant et retombent à l'état liquide dans la cartouche, immergeant progressivement celle-ci. Après immersion totale, le solvant chargé en extraits végétaux s'écoule par siphonage dans le ballon de départ. Le processus se poursuit en continu, le liquide dans le ballon étant de plus en plus chargé en extraits végétaux.

L'extraction est réalisée pendant 12 heures.

Les fractions hexaniques sont ensuite évaporées à sec sous pression réduite.

### EXEMPLE 1 : Lait corporel H/E

Ce lait a la composition suivante (% en poids) :

| | |
|---|---|
| - Stéarate de glycérol | 2 % |
| - Tween 60 (monostéarate de sorbitan à 20 moles d'oxyde d'éthylène) vendu par la Société ICI | 1 % |
| - Acide stéarique | 1,4 % |
| - Triéthanolamine | 0,7 % |
| - Carbopol 940 (neutralisé par de la triéthanolamine) | 0,2 % |
| - Huile d'amandes douces | 3 % |
| - Huile de vaseline | 8 % |
| - Extrait hydroalcoolique de noix d'Alep | 0,1 % |
| - Extrait de Ginkgo | 1 % |
| - Eau déminéralisée stérile + conservateurs qsp | 100 % |

H/E signifie : emulsion du type huile-dans-l'eau.

Le Carbopol 940 est un acide polyacrylique réticulé vendu par Goodrich.

On chauffe à 75-80°C le stéarate de glycérol, le Tween 60, l'acide stéarique, les huiles. On ajoute la triéthanolamine. Ce mélange est versé dans le Carbopol neutralisé en présence de 60 g d'eau. On abaisse la température à 40°C. On incorpore les extraits, le restant d'eau et les conservateurs.

### EXEMPLE 2 : Crème de soins pour le corps H/E

De façon analogue, on a préparé la crème suivante :

| | |
|---|---|
| - Stéarate de glycérol | 2 % |
| - Tween 60 (monostéarate de sorbitan à 20 moles d'oxyde d'éthylène) vendu par la Société ICI | 1 % |
| - Alcool cétylique | 0,5 % |
| - Acide stéarique | 1,4 % |
| - Triéthanolamine | 0,7 % |
| - Carbopol 940 (neutralisé par de la triéthanolamine) | 0,4 % |
| - Fraction liquide de graisse de karité | 12 % |
| - Perhydrosqualène de synthèse | 12 % |
| - Extrait hydroalcoolique de noix d'Alep | 0,1 % |
| - Extrait de Ginkgo | 1 % |
| - Eau déminéralisée stérile + conservateurs qsp | 100 % |

### EXEMPLE 3 : Lotion pour les mains

| | |
|---|---|
| - Extrait de Ginkgo | 1 % |
| - Extrait de thé vert | 0,1 % |
| - Lécinol S10 | 0,375% |
| - Generol 122 ES | 0,625% |
| - D5 (Cyclométhicone) vendu par la Société Dow | 2 % |
| - Glycérine | 10 % |
| - Parahydroxybenzoate de méthyle | 0,3 % |
| - Eau q.s.p. | 100 % |

Lécinol S10 est la dénomination commerciale d'une lécithine hydrogénée vendue par la Société Nikko.

Generol 122 ES est la dénomination commerciale d'un phytostérol oxyéthyléné à 5 moles d'oxyde d'éthylène vendu par la Société Henkel.

### EXEMPLE 4 : Dispersion vésiculaire

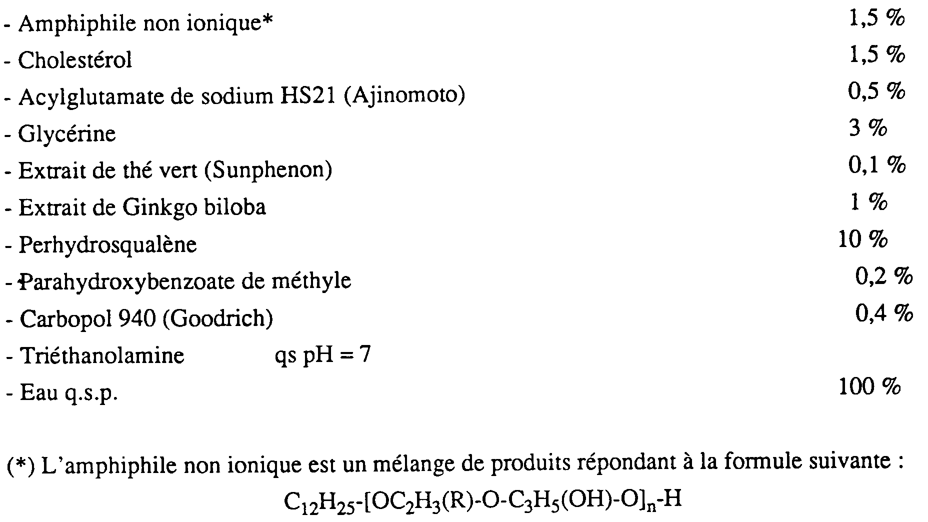

dans laquelle :
n, représentant le nombre statistique de motifs, est égal à 2,7,
les groupements -OC₂H₃(R)- représentent des radicaux : 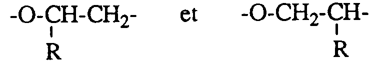 et les groupements -C₃H₅(OH)-O- représentent des radicaux : 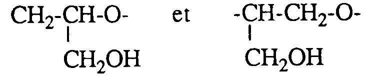 et les groupements R représentent un mélange équimolaire des radicaux C₁₄H₂₉ et C₁₆H₃₃.

Le produit vendu sous la dénomination "Acylglutamate HS21" est un stéarylglutamate disodique.

Cette dispersion vésiculaire est préparée de la façon suivante.

Le composé amphiphile non ionique est ajouté au cholestérol et à l'acylglutamate à une température de 100°C.

La température est abaissée à 90°C et on ajoute à cette température la glycérine, l'extrait de thé vert, l'extrait de Ginkgo et de l'eau (10 g).

Le mélange est refroidi à 50°C puis homogénéisé 2 fois 4 minutes à l'aide d'un homogénéiseur Virtis 60 (à 40.000 RPM).

Le produit obtenu est refroidi jusqu'à la température ambiante et dilué avec 20 g d'eau. La phase huileuse (perhydrosqualène et parahydroxybenzoate de méthyle) est ajoutée puis on homogénéise 2 fois 4 minutes à 40.000 RPM.

Le gel de Carbopol (Carbopol 940 et eau qsp 100) est dispersé pendant 30 secondes à 10.000 RPM puis l'ensemble est neutralisé par la triéthanolamine.

On obtient une crème lisse et brillante. Elle est utilisée sur le cuir chevelu pour protéger les cheveux des effets nocifs dus aux radicaux libres.

### ETUDE DE L'ACTIVITE ANTIOXYDANTE

L'efficacité du système antioxydant selon l'invention est démontré par la méthode d'oxydation accélérée de la vitamine F qui est une substance particulièrement sensible à l'oxydation.

Pour l'étude, on utilise le dispositif automatique "RANCIMAT" de la Société METROHM (A. Seher et al, Fette, Seifen, Anstrichmittel 88(1) 1-6, 1986).

On prépare des mélanges de vitamine F avec un extrait de thé vert "SUNPHENON" seul, avec un extrait hexanique de ginkgo seul, et avec un mélange d'un extrait de thé vert avec un extrait hexanique de ginkgo.

On porte chaque échantillon à 100°C, sous un barbotage d'air (20 litres/h). On suit alors en continu la concentration en acides volatils résultant de la dégradation des hydroperoxydes et des aldéhydes de vitamine F dans une cellule remplie d'eau dans laquelle on plonge une électrode en platine. Cette électrode mesure, en fonction du temps, l'augmentation de la conductivité provoquée par l'augmentation de la concentration d'acides volatils. Le temps d'induction sera déterminé par l'intersection des deux asymptotes de la courbe d'oxydation exponentielle obtenue.

Ce temps correspond au temps de latence précédant l'auto-oxydation de la vitamine F. Plus ce temps de latence est long, meilleure est la résistance de la vitamine F à l'auto-oxydation.

Les résultats sont les suivants :

| **Produit testé** | **Temps d'induction** |
|---|---|
| Thé Vert 0,1 % | 33 min |
| Ginkgo 1 % | 48 min |
| Thé Vert 0,1 % + Ginkgo 1 % | 258 min |

## Revendications

1. Composition cosmétique ou pharmaceutique, caractérisée par le fait qu'elle contient un système antioxydant à action synergique constitué par l'association d'un extrait de ginkgo et d'au moins un composé polyphénolique, ledit extrait de ginkgo pouvant être obtenu par extraction de feuilles de ce végétal à l'aide d'un solvant non polaire.

2. Composition selon la revendication 1, caractérisée par le fait que ledit solvant non polaire est un alcane en C₆-C₁₄.

3. Composition selon la revendication précédente, caractérisée par le fait que ledit solvant non polaire est le n-hexane.

4. Composition selon l'une quelconque des revendications précédentes, caractérisée par le fait que ledit composé polyphénolique comporte au moins un cycle aromatique diphénolique, les groupements phénols pouvant être éventuellement éthérifiés ou estérifiés.

5. Composition selon l'une quelconque des revendications précédentes, caractérisée par le fait que ledit composé polyphénolique est choisi parmi les flavonoïdes, l'acide carnosique, le carnosol, les acides (2,5-dihydroxyphényl)carboxylique et (2,5-dihydroxy phényl)alkylène carboxyliques essentiellement substitués, et leurs dérivés, les esters ou amides d'acide caféique et l'acide tannique.

6. Composition selon la revendication 5, caractérisée par le fait que lesdits flavonoïdes sont des flavonoïdes extraits de feuilles de thé.

7. Composition selon la revendication 5, caractérisée par le fait que lesdits flavonoïdes sont choisis parmi la taxifoline, la catéchine, l'épicatéchine, l'ériodictyol, la naringénine, la rutine, la troxérutine, la chrysine, la tangérétine, la lutéoline, l'épigallocatéchine et le gallate d'épigallocatéchine, la quercétine, la fisétine, le kaëmpférol, la galangine, la gallocatéchine et le gallate d'épicatéchine.

8. Composition selon l'une quelconque des revendications précédentes, caractérisée par le fait que l'extrait de ginkgo est présent à une concentration comprise entre 1 et 10 % en poids par rapport au poids total de la composition.

9. Composition selon l'une quelconque des revendications précédentes, caractérisée par le fait que le composé polyphénolique est présent en une proportion comprise entre 0,1 et 1 % en poids par rapport au poids total de la composition.

10. Composition selon l'une quelconque des revendications précédentes, caractérisée par le fait que le rapport pondéral de l'extrait de ginkgo au composé polyphénolique est compris entre 1 et 100 et en particulier voisin de 10.

11. Utilisation en association, d'au moins un extrait de ginkgo et d'au moins un composé polyphénolique comme association active synergique dans la préparation d'une composition cosmétique ou pharmaceutique destinée à prévenir ou traiter les dommages cellulaires provoqués, sur la peau, le cuir chevelu ou les muqueuses, par les radicaux libres induits notamment par les polluants atmosphériques et/ou par le rayonnement ultraviolet, et/ou destinée à lutter contre le phénomène de vieillissement accéléré de la peau.

12. Utilisation selon la revendication précédente, caractérisée par le fait que ladite composition présente les caractéristiques telles que définies dans l'une quelconque des revendications 2 à 10.

13. Procédé de traitement cosmétique permettant de lutter contre les dommages esthétiques provoqués sur la peau et les cheveux par les radicaux libres induits notamment par les polluants atmosphériques et par le rayonnement ultraviolet, caractérisé par le fait que l'on applique sur la peau ou les cheveux une composition contenant l'association synergique d'un extrait de ginkgo et d'un composé polyphénolique.

14. Procédé selon la revendication précédente, caractérisé par le fait que ladite composition présente les caractéristiques telles que définies dans l'une quelconque des revendications 2 à 10.

## Claims

1. Cosmetic or pharmaceutical composition, characterized in that it contains an antioxidizing system possessing a synergistic action consisting of the combination of a ginkgo extract and at least one polyphenol compound, it being possible to obtain the said ginkgo extract by extracting leaves of this plant using a nonpolar solvent.

2. Composition according to Claim 1, characterized in that said nonpolar solvent is a C₆-C₁₄ alkane.

3. Composition according to the preceding claim, characterized in that the said nonpolar solvent is n-hexane.

4. Composition according to any one of the preceding claims, characterized in that the said polyphenol compound contains at least one diphenol aromatic ring, it being possible for the phenol groups to be optionally etherified or esterified.

5. Composition according to any one of the preceding claims, characterized in that the said polyphenol compound is chosen from flavonoids, carnosic acid, carnosol, optionally substituted 2,5-dihydroxybenzoic and (2,5-dihydroxyphenyl)alkylenecarboxylic acids and their derivatives, esters or amides of caffeic acid and tannic acid.

6. Composition according to Claim 5, characterized in that the said flavonoids are flavonoids extracted from tea leaves.

7. Composition according to Claim 5, characterized in that the said flavonoids are chosen from taxifolin, catechin, epicatechin, eriodictyol, naringenin, rutin, troxerutin, chrysin, tangeretin, luteolin, epigallocatechin and epigallocatechin gallate, quercetin, fisetin, kaempferol, galangine, gallocatechin and epicatechin gallate.

8. Composition according to any one of the preceding claims, characterized in that the ginkgo extract is present at a concentration of between 1 and 10% by weight with respect to the total weight of the composition.

9. Composition according to any one of the preceding claims, characterized in that the polyphenol compound is present in a proportion of between 0.1 and 1% by weight with respect to the total weight of the composition.

10. Composition according to any one of the preceding claims, characterized in that the ratio by weight of the ginkgo extract to the polyphenol compound is between 1 and 100 and in particular in the region of 10.

11. Use of a combination of at least one ginkgo extract and at least one polyphenol compound, as synergistic active combination, in the preparation of a cosmetic or pharmaceutical composition intended to prevent or treat cell damage caused, on the skin, scalp or mucous membranes, by free radicals induced especially by atmospheric pollutants and/or by ultraviolet radiation, and/or intended to control the phenomenon of accelerated skin ageing.

12. Use according to the preceding claim, characterized in that the said composition has the characteristics as defined in any one of Claims 2 to 10.

13. Process for cosmetic treatment which makes it possible to control damage, giving an unpleasant appearance to the skin and hair, caused by free radicals induced especially by atmospheric pollutants and by ultraviolet radiation, characterized in that a composition containing the synergistic combination of a ginkgo extract and a polyphenol compound is applied to the skin or the hair.

14. Process according to the preceding claim, characterized in that the said composition has the characteristics as defined in any one of Claims 2 to 10.

## Patentansprüche

1. Kosmetische oder pharmazeutische Zubereitung, dadurch gekennzeichnet, daß sie ein antioxidatives System mit synergistischer Wirkung enthält, welches aus der Kombination eines Ginko-Extrakts mit mindestens einer polyphenolischen Verbindung besteht, wobei der Ginko-Extrakt durch Extraktion von Blättern dieser Pflanze mit Hilfe eines apolaren Lösungsmittles erhalten werden kann.

2. Zubereitung gemäß Anspruch 1, dadurch gekennzeichnet, daß das apolare Lösungsmittel ein C₆-C₁₄-Alkan ist.

3. Zubereitung gemäß dem vorstehenden Anspruch, dadurch gekennzeichnet,daß das apolare Lösungsmittel n-Hexan ist.

4. Zubereitung gemäß einem der vorstehenden Ansprüche, dadurch gekennzeichnet, daß die polyphenolische Verbindung mindestens einen diphenolischen, aromatischen Ring trägt, wobei die Phenolgruppen gegebenenfalls verethert oder verestert sein können.

5. Zubereitung gemäß einem der vorstehenden Ansprüche, dadurch gekennzeichnet, daß die polyphenolische Verbindung ausgewählt ist unter den Flavonoiden, Karnosinsäure, Karnosol, im wesentlichen substituierten 2,5-Dihydroxyphenylcarbonsäuren und 2,5-Dihydroxyphenylalkylcarbonsäuren sowie deren Derivaten, Estern oder Amiden, Kaffeesäure oder Tannin.

6. Zubereitung gemäß Anspruch 5, dadurch gekennzeichnet, daß es sich bei den Flavonoiden um aus Teeblättern extrahierte Flavonoide handelt.

7. Zubereitung gemäß Anspruch 5, dadurch gekennzeichnet, daß die Flavonoide ausgewählt sind unter Taxifolin, Catechin, Epicatechin, Eriodictyol, Naringenin, Rutin, Troxerutin, Chrysin, Tangeretin, Luteolin, Epigallocatechin und Epigallocatechingallat, Quercetin, Fisetin, Kämpferol, Galangin, Gallocatechin und Epicatechingallat.

8. Zubereitung gemäß einem der vorstehenden Ansprüche, dadurch gekennzeichnet, daß der Ginko-Extrakt in einer Konzentration zwischen 1 und 10 Gew.-%, bezogen auf das Gesamtgewicht der Zubereitung, vorhanden ist.

9. Zubereitung gemäß einem der vorstehenden Ansprüche, dadurch gekennzeichnet, daß die polyphenolische Verbindung in einer Menge zwischen 0,1 und 1 Gew.-%, bezogen auf das Gesamtgewicht der Zubereitung, vorhanden ist.

10. Zubereitung gemäß einem der vorstehenden Ansprüche, dadurch gekennzeichnet, daß das Gewichtsverhältnis von Ginko-Extrakt zur polyphenolischen Verbindung zwischen 1 und 100 und insbesondere in der Nähe von 10 liegt.

11. Verwendung einer Kombination mindestens eines Ginko-Extrakts mit mindestens einer polyphenolischen Verbindung als synergistische wirkende Kombination bei der Herstellung einer kosmetischen oder pharmazeutischen Zubereitung, die zur Vorbeugung oder Behandlung von Zellschäden, die auf der Haut, der Kopfhaut oder den Schleimhäuten von insbesondere durch Umweltschadstoffe und/oder ultraviolette Bestrahlung induzierten freien Radikalen hervorgerufen werden, und/oder zur Bekämpfung des Phänomens der vorzeitigen Hautalterung bestimmt sind.

12. Verwendung gemäß dem vorstehenden Anspruch, dadurch gekennzeichnet, daR die Zubereitung Eigenschaften aufweist, wie sie in einem der Ansprüche 2 bis 10 definiert sind.

13. Verfahren zur kosmetischen Behandlung, welches die Bekämpfung von ästhetischen Schäden gestattet, die auf der Haut und der Kopfhaut von freien Radikalen hervorgerufen werden, die insbesondere von Umweltschadstoffen und durch ultraviolette Bestrahlung induziert werden, dadurch gekennzeichnet, daß man auf die Haut oder die Haare eine Zubereitung appliziert, die eine synergistische Kombination eines Ginko-Extrakts mit einer polyphenolischen Verbindung enthält.

14. Verfahren gemäß dem vorstehenden Anspruch, dadurch gekennzeichnet, daR die Zubereitung Eigenschaften aufweist, wie sie in einem der Ansprüche 2 bis 10 definiert sind.
